# EUROPEAN PATENT APPLICATION

(11) **EP 4 008 320 A1**
(43) Date of publication of application: **08.06.2022**
(21) Application number: 20851097.4
(22) Date of filing: 31.07.2020
(51) Int. Cl.: A61K 9/48, G06K 19/04, G06K 19/06, A61J 3/07

(54) **HARD CAPSULE FORMULATION SEALED WITH BAND SEAL INCLUDING TAG**

(30) Priority: 02.08.2019 JP 2019143115
(71) Applicant: Qualicaps Co., Ltd., Nara 639-1032 (JP)
(72) Inventor: KATO, Nobuhiro, Yamatokoriyama-shi, Nara 639-1032 (JP); HONDA, Mamoru, Yamatokoriyama-shi, Nara 639-1032 (JP)
(74) Representative: Müller-Boré & Partner Patentanwälte PartG mbB
(86) International application number: PCT/JP2020/029380
(87) International publication number: WO 2021/024930

(57) **Abstract**

There is provided a method by which microtags can be attached to a product at a product processing facility other than the manufacturer such as a dispensing pharmacy. Also, it is an object to attach microtags to a hard capsule in a simple manner. There is provided a band seal preparation liquid for sealing a hard capsule, the band seal preparation liquid containing microtags that enable a product to be identified.

## Description

### Technical Field

Disclosed in this description are a hard capsule sealed with a band seal containing tags, a band seal preparation liquid containing tags, a method for preparing a hard capsule sealed with a band seal containing tags, a method for preventing counterfeit of hard capsules using tags, and a method for tracking hard capsules using tags.

### Background Art

In the field of medicines and foods, after a maker manufactures a legitimate medicine or supplement, a third party may sometimes modify the product.

As a method for monitoring such post-manufacture modification of a product, Patent Literature 1 and Patent Literature 2 describe tracer particles for tracking products and tagging products with the tracer particles to track the products after manufacture. In Patent Literature 1, the tracer particles are used to tag medicines, animal feeds and so on. This product is sold under the name SECURtracers by Micro-Tracers, Inc. (San Francisco, California, USA) (Non-Patent Literature 1, Non-Patent Literature 2). Also, Patent Literature 2 discloses using a porous silicon microtag as a label for a product. The label described in Patent Literature 2 is sold under the name TruTag (trademark) microtag by TruTag Technologies, Inc. (Kapolei, Hawaii, USA) (Non-Patent Literature 3).

### Citation List

### Patent Literature

Patent Literature 1:US Patent No. 10,175,199
Patent Literature 2:US Patent No. 10,078,766

### Non-Patent Literature

Non-Patent Literature 1:Web page https://www.securtracers.com/aboutsecurtracers
Non-Patent Literature 2:Web page https://www.securtracers.com/applications
Non-Patent Literature 3:Web page https://trutags.com/pharmaceutical-nutraceutical/

### Summary of Invention

### Technical Problem

In Patent Literature 1 and Patent Literature 2, it is described that the tracer particles are introduced into an entire drug to tag the products by mixing the tracer particles into a solid excipient used in manufacturing tablets or mixing the tracer particles into a liquid excipient used in manufacturing a liquid formulation. Also, Patent Literature 1 and Patent Literature 2 describe a method for attaching the tracer particles to the entire surfaces of tablets by mixing the tracer particles into a coating liquid for the tablets. The method in which tracer particles are mixed into entire drugs or a coating is applied to entire drugs requires a step of attaching the tracer particles to the products to be performed on the side of the manufacturer of medicines, supplements or the like.

Also, in the method described in Non-Patent Literature 3 as well, it is necessary to attach the microtags directly to products such as medicines and supplements.

However, in recent years, at dispensing pharmacies or the like, drugs are prescribed by purchasing a bulk material of an active ingredient of a drug or the like and packaging the active ingredient into single doses in the dispensing pharmacies. With the methods described in Patent Literature 1, Patent Literature 2 and Non-Patent Literature 3, it is difficult to tag products with tracer particles or microtags at dispensing pharmacies, and it is therefore difficult to track the dispensing pharmacy where the product was packaged into single doses.

In view of such problems, it is an object of the present invention to provide a method by which it is possible to attach tags to products even in a product processing facility other than the manufacturer such as a dispensing pharmacy.

Also, Non-Patent Literature 2 describes adding tracer particles onto a capsule band. However, when tracer particles are added onto a capsule band, it is necessary to use another solution to attach the tracer particles onto a band seal of an original capsule. Thus, it is complicated to prepare a solution containing tracer particles that is different from the band seal preparation liquid.

In view of such a problem, it is an object of the present invention to attach tags to hard capsules in a simpler manner.

### Solution to Problem

As a result of intensive studies, the present inventors found that tags can be detected even without dissolving a hard capsule when tags are dispersed in a band seal preparation liquid used in sealing hard capsules and this band seal preparation liquid is used to seal hard capsules.

The present invention has been made based on this finding, and includes the following embodiments.

Embodiment 1. A hard capsule formulation sealed with a band seal preparation liquid containing tags that enable a product to be identified.

Embodiment 2. The hard capsule formulation according to Embodiment 1, in which the tags have a length of 400 µm or less.

Embodiment 3. The hard capsule formulation according to Embodiment 1 or 2, in which the tags have an identifier for identifying the product.

Embodiment 4. The hard capsule formulation according to Embodiment 3, in which the identifier can be read when irradiated with light in a predetermined wavelength region.

Embodiment 5. The hard capsule formulation according to Embodiment 3 or 4, in which the identifier is at least one selected from the group consisting of letters, colors, figures, trademarks, emblems, one-dimensional barcodes, two-dimensional barcodes, and rugate type structures.

Embodiment 6. The hard capsule formulation according to any one of Embodiments 1 to 5, in which a film of the hard capsule contains a base and a component other than the base, the base being at least one selected from the group consisting of gelatin, cellulose compounds, polyvinyl alcohol, polyvinyl alcohol copolymers and pullulan, the component other than the base being at least one selected from gelling agents, gelation aids, plasticizers, lubricants, metal sequestering agents, coloring agents, light-shielding agents, and residual moisture.

Embodiment 7. A band seal preparation liquid for sealing a hard capsule, the band seal preparation liquid containing tags that enable a product to be identified.

Embodiment 8. The band seal preparation liquid according to Embodiment 7, having a specific gravity that allows tags to be dispersed for a predetermined period of time.

Embodiment 9. A method for preparing a hard capsule formulation, comprising filling a hard capsule with a content, followed by fitting a cap portion and a body portion thereof together, applying a band seal preparation liquid according to Embodiment 7 or 8 to a formed fitted portion, and drying the band seal preparation liquid to seal the hard capsule.

Embodiment 10. A method for preventing counterfeit of a hard capsule formulation, comprising filling a hard capsule with a content, followed by fitting a cap portion and a body portion thereof together, applying a band seal preparation liquid according to Embodiment 7 or 8 to a formed fitted portion, and drying the band seal preparation liquid to seal the hard capsule.

Embodiment 11. A method for tracking a hard capsule formulation, comprising a step of irradiating a hard capsule formulation according to any one of Embodiments 1 to 6 with light in a predetermined wavelength region to detect the tags or identifier.

### Advantageous Effects of the Invention

According to the present invention, it is possible to attach tags to a hard capsule formulation at a product processing facility or the like other than the manufacturer. Also, it is possible to reduce the amount of tags to be attached to capsule products by adding tags to a band seal. Further, detection is easy because there is no need to separate the tags from other components when the tags are detected.

### Brief Description of Drawings

FIG. 1 shows a method for sealing a hard capsule.
FIG. 2 shows a schematic view of a tagged hard capsule.
FIG. 3 shows images of a capsule sealed with a band seal described in Example 1 obtained under a fluorescent lamp (left) and under B excitation light.
FIG. 4 shows a fluorescent microscope image (B excitation) of a sealed portion of a capsule sealed with a band seal described in Example 1.
FIG. 5 shows images of a capsule sealed with a band seal described in Example 2 obtained under a fluorescent lamp (left) and under B excitation light.
FIG. 6 shows a fluorescent microscope image (B excitation) of a sealed portion of a capsule sealed with a band seal described in Example 2.

### Description of Embodiments

### 1. Description of terms

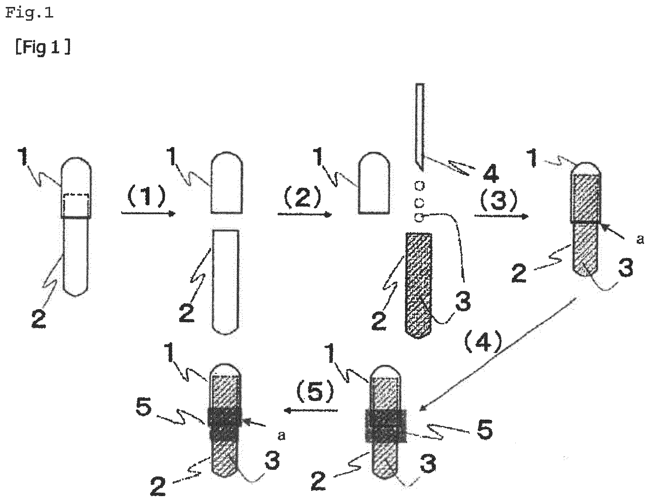

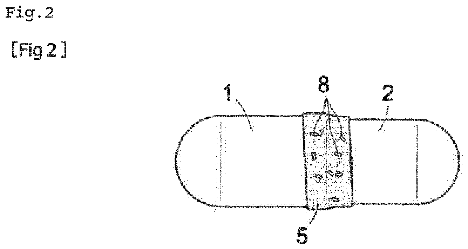

In this description, a "band seal" means a band-like sealing film (reference numeral 5) formed on a hard capsule formulation prepared by combining a body portion (reference numeral 2) and a cap portion (reference numeral 1) as shown in FIG. 1 by applying a band seal preparation liquid to an outer peripheral surface of the body portion (reference numeral 2) and an outer peripheral surface of the cap portion (reference numeral 1) with a cut end (end edge) (reference numeral a) of the cap portion generally in the middle at a width across it one to several times in a circumferential direction, and drying the band seal preparation liquid. Also, FIG. 2 shows a schematic view of a tagged hard capsule. The hard capsule shown in FIG. 2 has been sealed with a band seal (reference numeral 5) containing tags (reference numeral 8).

In this description, a "band seal preparation liquid" means a solution for forming the band seal, in other words, a solution of a component that will form a band seal (which is hereinafter also referred to as "seal component") dissolved in a solvent such as water or ethanol. The seal component can be selected based on a component of a base (which is hereinafter also referred to as "base component") of a film of a hard capsule described later (which is hereinafter also referred to simply as "film"). The seal component may be the same as or different from the base component of the film. In general, the seal component contains the base component of the film to which the band seal will be applied. For example, when gelatin is used as the base component of the film, gelatin can be used as the seal component. Also, when hydroxypropylmethylcellulose (which is also referred to as HPMC or hypromellose) as a cellulose compound is used as the base component of the film, hydroxypropylmethylcellulose can be used as the seal component. Examples of cellulose compounds other than hydroxypropylmethylcellulose can include ethylcellulose, hydroxypropylcellulose, low-substituted hydroxypropylmethylcellulose, hydroxypropylcellulose, methylcellulose, hydroxyethylcellulose, hydroxyethylmethylcellulose, hydroxyethylethylcellulose, hydroxypropylmethylcellulose phthalate, hydroxypropylmethylcellulose acetate succinate, and cellulose acetate phthalate. Also, examples of other base components can include pullulan, polyvinyl alcohol (PVA), polyvinyl alcohol (PVA) copolymers and mixtures of PVA and a PVA copolymer, and methacrylic acid copolymers. As a band seal preparation liquid containing a cellulose compound, a band seal preparation liquid described in, for example, Japanese Unexamined Patent Application Publication No. 2005-187412 can be exemplified. As band seal preparation liquids containing PVA, a PVA copolymer and a mixture of PVA and a PVA copolymer, band seal preparation liquids described in, for example, WO2008/099835 can be exemplified.

The band seal preparation liquid can use water, a mixed liquid of water and ethanol, a mixed liquid of water and isopropanol or the like as a solvent. The solvent can be selected according to the material of the base as well as the material of the substrate (base) of the tags.

The band seal preparation liquid may contain, in addition to the seal component, additives or the like usually used for preparation of a hard capsule film, such as plasticizers, coloring agents, opacifying agents or fragrances, in such a range that the formation of a band seal is not inhibited. The additives can be selected based on the seal component and the base component of the hard capsule film to which the band seal will be applied. For example, when gelatin is used as the seal component, polysorbate 80 can be used as a plasticizer.

Also, a solution containing a component other than the base component of the film to which the band seal will be applied as a seal component can be used as a band seal preparation liquid. For example, a band seal preparation liquid containing gelatin as a seal component may be applied to a hard capsule using hydroxypropylmethylcellulose as the base component of the film thereof. Also, as a band seal preparation liquid, a band seal preparation liquid containing monoacetyl monoacylglycerin as a seal component as described in Japanese Unexamined Patent Application Publication No. 2015-218131, a band seal preparation liquid containing a plant polysaccharide thickener having low or no gel-forming ability but having film-forming properties, such as pullulan, starch or cellulose, as a seal component as described in Japanese Unexamined Patent Application Publication No. 2006-22028, or a band seal preparation liquid containing one or two or more of (a) a polyvinylpyrrolidone with a molecular weight of 100,000 to 4,000,000, (b) a polyvinylpyrrolidone with a molecular weight of 10,000 to 80,000 that is 90% by weight or less based on the total weight of the band seal, and (c) a copolymer of 1-vinyl -2-pyrrolidone and vinyl acetate as a seal component as described in WO2006/070578, for example, may be used.

The content of the seal component in the band seal preparation liquid is not limited as long as a band seal can be formed. For example, the content of the base can be selected from the range of 10% (mass/mass) to 40% (mass/mass) when the dosage of the band seal preparation liquid is set to be 100 g. Also, the content of the components other than the seal component can be 0% (mass/mass) or more when the dosage of the band seal preparation liquid is set to be 100 g, and can be selected from the range up to about 5% (mass /mass). In order to prevent dripping or the like when the band seal preparation liquid is applied, the viscosity of the band seal preparation liquid may usually be adjusted such that the preparation liquid will have a final viscosity in the range of 100 to 5,000 mPa·s. It should be noted that the viscosity specified in the present invention is intended to mean a viscosity measured with a B-type rotational viscometer, using No. 2 rotor when the viscosity is less than 500 mPa·s, No. 3 rotor when the viscosity is 500 mPa·s or more and less than 2,000 mPa·s, or No. 4 rotor when the viscosity is 2,000 mPa·s or more, under the conditions of 23°C, a rotational speed of 12 rpm, and a measurement time of 1 minute.

In this description, the band seal preparation liquid contains tags.

The tags are not limited as long as products to which the tags have been attached can be identified. The products can be identified based on the presence or absence of the tags or an identifier the tags have.

The tags are also referred to as tracer particles in Patent Literature 1 and Patent Literature 2. Also, in Patent Literature 3, the tags are also referred to as porous silicon microtags. Also, as described in Patent Literature 1 and Patent Literature 2, the tags may be also referred to as microtags, taggants, markers and/or fine particles. Further, the tags may be also referred to as identification (ID) tags.

The size of the tags is not limited as long as they have a size that allows them to be attached to tablets, capsules or the like and does not affect the recipients' swallowing. Also, the size can be set based on the size of the identifier to be attached to the tags. The size of the tags is preferably on a micrometer scale.

The tags may be in the form of a plate or film. Preferably, the tags may be produced by applying a detection reagent to plate-like or film-like bases.

The shape of the tags can be selected from a substantially quadrangular shape, a substantially triangular shape, a substantially circular shape, a substantially elliptical shape, a substantially polygonal shape with five or more corners, and so on.

The tags have a length of, for example, 400 µm or less, more preferably 300 µm or less. The tags have a width of, for example, 100 µm or less, preferably 50 µm or less, more preferably 10 µm or less. Even when the width is 5 µm or less, an identifier can be attached. In the present invention, one object is to simplify the detection of the tags. Thus, a width of less than 1 µm is not preferred because the identifier cannot be read without the use of a high-resolution microscope. Here, when the tags have a generally quadrangular shape, the length is the length of their long sides and the width is the length of their short sides if the generally quadrangular shape is assumed to be a quadrangular shape. When the tags have a generally triangular shape, the length is the length of their bases and the width is the length of a line segment that is perpendicular to the base and represents the height of the triangle if the generally triangular shape is assumed to be a triangular shape. When the tags have a generally circular shape, the length and width are their diameter if the generally circular shape is assumed to be a circular shape. When the tags have a generally elliptical shape, the length is that of their major axes and the width is that of their minor axes if the generally elliptical shape is assumed to be an elliptical shape. When the tags have a generally polygonal shape, the length is the length of a line segment extending through the center of gravity of the generally polygonal shape and located between the intersections with sides of the generally polygonal shape. When the tags have a generally polygonal shape, the width is the length of the longest line segment among line segments extending perpendicular to the line segment representing the length and located between the intersections with sides of the generally polygonal shape.

The substrate (base) of the tags is not limited as long as the tags with a shape as described above can be formed. The base of the tags is preferably a material acceptable to be used in medicines or supplements that are orally ingested.

More specifically, examples of the raw material for the base of the tags can include cellulose compounds such as ethylcellulose, hydroxypropylmethylcellulose, hydroxypropylcellulose, methylcellulose, hydroxypropylmethylcellulose phthalate, hydroxypropylmethylcellulose acetate succinate, and cellulose acetate phthalate; gelatin; pullulan; PVA; PVA copolymers, silicon, and mixtures thereof.

The tags preferably have an identifier. The identifier attached to the base is not limited as long as it is information that directly or indirectly gives a discriminatory effect to humans, such as letters, figures, colors (including fluorescent emission), trademarks, emblems, one-dimensional barcodes, two-dimensional barcodes, and rugate type structures. A direct discriminatory effect means that a human can grasp the meaning of the identifier upon observing it through a magnifier or the like, that the identifier can be recognized to contain a substance that fluoresces when irradiated with a specific wavelength, and that an identifier visualized by fluorescence can be recognized. For example, letters, trademarks, emblems and so on can be exemplified as such identifiers. Also, an indirect discriminatory effect means that a human can grasp the information that is recorded as the identifier by reading the identifier with a reading device or the like and converting the information of the identifier on a computer. For example, one-dimensional barcodes, two-dimensional barcodes, rugate type structures and so on can be exemplified as such identifiers. The identifier may contain not only meaningful information but also information such as a mere row of letters, symbols or figures the sequence of which itself is meaningless.

The identifier used may be composed of one letter or a plurality of letters. For example, as the letters, the company name or facility name of the manufacturer or formulation processing facility of the product such as a medicine or supplement or an abbreviated name thereof; a trademark; the name of the drug to which the tags are attached and its active ingredient name or an abbreviated name thereof; the dosage of the drug or active ingredient and so on can be exemplified.

As the figures, the symbol mark of the company or facility of the manufacturer or formulation processing facility of the product such as a medicine or supplement, a trademark, the symbol mark of the product and so on can be exemplified.

The trademarks may include a combination of letters and figures as well as those composed of only letters or only figures. The trademarks are intended to indicate the origin of the product or to identify the product.

The emblems may include a combination of letters and figures as well as those composed of only letters or only figures. The emblems are not intended to indicate the origin of the product or the like but are intended to indicate marks or the like indicating, for example, specifications or technical standards.

As the one-dimensional barcodes, JAN (Japanese Article Number), NW-7 (CODABAR), Code 2 of 5 (Industrial 2 of 5), ITF (INTERLEAVED 2 of 5), Code 39, Code 93, Code 128, GS1 DataBar (RSS) and so on can be exemplified.

As the two-dimensional barcodes, QR code (registered trademark), micro QR code, DataMatrix, MaxiCode, PDF417, MicroPDF417 and so on can be exemplified.

The identifiers with a rugate type structure are exemplified in Patent Literature 3.

The method for attaching the identifier to the tags is not limited. For example, a positive or negative photoresist method, a microcontact printing method, a method described in Patent Literature 3 and so on can be exemplified.

For example, in the microcontact printing method, a stamp is initially made by taking an impression of a master of the identifier with a silicone rubber (PDMS). A solution of a detection reagent that contains a detection substance containing at least one selected from magnetic substances such as iron powder, reflective substances such as titanium dioxide, fluorescent substances and so on, and the component of the base or a component similar thereto is applied to one side or both sides of a base and dried. Then, a component that can dissolve the base or the component similar thereto contained in the detection reagent is applied to the stamp, and the stamp is pressed against the base to which the detection reagent has been applied to dissolve part of the detection reagent. For example, in the method described in Patent Literatures 1 and 2, ethylcellulose is used as the base, and an identifier is attached by pressing a stamp to which ethanol has been applied against the base to which a detection reagent containing ethylcellulose has been applied to dissolve part of the dried detection reagent. The dissolution of the detection reagent is preferably applied to both sides of the tags.

The tags preferably have a specific gravity that is similar to or slightly higher than the specific gravity of the band seal preparation liquid. Preferably, the specific gravity of the tags is about 0.1 to 300% higher than the specific gravity of the band seal preparation liquid. With such a configuration, the tags can remain dispersed for a predetermined period of time after the tags are dispersed in the band seal preparation liquid. Such a property is advantageous because it allows a band seal preparation liquid containing the tags to remain set in a sealing machine for a certain period of time. The predetermined period of time is from about one hour to eight hours, preferably from about three hours to six hours. The specific gravity of the band seal preparation liquid can be measured according to "6 Methods of measuring density and specific gravity with a specific gravity bottle" described in JIS Z 8804:2012, for example. For the specific gravity of the tags, reference can be made to the specific gravity of the base, for example.

The amount of tags to be added to the band seal preparation liquid can be 0.01% (mass/mass) to 2.0% (mass/mass). By adding tags to the band seal preparation liquid, the amount of tags to be used can be reduced.

The detection substance preferably emits reflected light or fluoresces when irradiated with light in a predetermined wavelength region. With such a configuration, the tags themselves can be detected or the identifier attached to the tags can be read. As the light in a predetermined wavelength region, light in a visible region, or light in an ultraviolet region can be exemplified. As the light in a visible region, about 380 nm to 750 nm can be exemplified. As the light in a visible region, preferred is light in a blue region between 460 nm and 495 nm. The light in a predetermined wavelength region can be selected according to the detection substance, and/or the color of the hard capsule or the color of the band seal.

As the tags, SECURtracers (Micro-Tracers, Inc., San Francisco, California, USA), or TruTag (trademark) microtags (TruTag Technologies, Inc., Kapolei, Hawaii, USA) may be used.

The "hard capsule" to which a band seal preparation liquid containing tags that is disclosed in this description may be applied is a capsule of the type in which a capsule film is initially produced and a content is filled into the produced capsule film. It is usually composed of a cap portion and a body portion, and also called hard capsule or two-piece capsule. A soft capsule produced by filling a content between two films and bonding the films to each other, a seamless capsule produced by dripping a content together with a film solution into a solidification liquid, and a microcapsule prepared by introducing an active ingredient into the inside thereof by precipitation or emulsification of a base are not included in the "hard capsule" of the present invention.

The film forming the hard capsule (which is also referred to as "hard capsule film") may contain a base component and components other than the base.

The hard capsule film may be gastrosolble or enteric. The base component of the hard capsule film is not limited. As the base component of the hard capsule film, gelatin, pullulan, cellulose compounds, PVA, PVA copolymers, methacrylic acid copolymers and so on can be exemplified. Examples of the cellulose compounds can include ethylcellulose, hydroxypropylmethylcellulose, low-substituted hydroxypropylmethylcellulose, hydroxypropylcellulose, methylcellulose, hydroxyethylcellulose, hydroxyethylmethylcellulose, hydroxyethylethylcellulose, hydroxypropylmethylcellulose phthalate, hydroxypropylmethylcellulose acetate succinate, and cellulose acetate phthalate, methacrylic acid copolymers and so on.

As the components other than the base component contained in the hard capsule film, at least one selected from gelling agents, gelation aids, plasticizers, lubricants, metal sequestering agents, coloring agents and light-shielding agents acceptable as pharmaceutical and food additives, and residual moisture may be contained.

As the gelling agent, carrageenan, tamarind seed polysaccharide, pectin, xanthan gum, locust bean gum, curdlan, gelatin, furcellaran, agar, gellan gum and so on can be exemplified. These may be used singly in one kind or in any combination of two or more thereof.

Among the above gelling agents, carrageenan is the most suitable gelling agent as it has high gel strength, and exhibits excellent gelation properties under the coexistence with specific ions even when used in a small amount. It should be noted that, in general, three types of carrageenan are known as carrageenan; kappa-carrageenan, iota-carrageenan and lambda-carrageenan. In the present invention, kappa- and iota-carrageenans, which have an ability to form a gel with relatively high hardness, can be suitably used. Also, pectin can be classified into LM pectin and HM pectin according to the difference in the degree of esterification, and gellan gum can be also classified into acylated gellan gum (native gellan gum) and deacylated gellan gum according to the presence or absence of acylation. Any of them, however, can be used with no distinction.

A gelation aid can be selected according to the type of the gelling agent used. When carrageenan is used as a gelling agent, examples of gelation aids that can be used in combination therewith are as follows. For kappa-carrageenan, example can include compounds capable of providing one or two or more of potassium ions, ammonium ions and calcium ions in water, such as potassium chloride, potassium phosphate, ammonium chloride, ammonium acetate, and calcium chloride. For iota-carrageenan, examples can include compounds capable of providing calcium ions in water, such as calcium chloride. When gellan gum is used as a gelling agent, examples of gelation aids that can be used in combination therewith can include compounds capable of providing one or two or more of sodium ions, potassium ions, calcium ions and magnesium ions in water, such as sodium chloride, potassium chloride, calcium chloride, and magnesium sulfate. In addition, citric acid or sodium citrate can be also used as an organic acid or a water-soluble salt thereof.

When the base of the hard capsule film is hydroxypropylmethylcellulose, it is preferred to use a gelling agent and a gelation aid.

A Plasticizer, a surfactant (emulsifying agent), a base (except for a nonionic water-soluble cellulose compound), a binder (except for PVA), a coating agent or the like acceptable as a pharmaceutical and food additive may be contained. Also, a release-prolonging agent, a dissolution aid, a solubilizing agent or the like for controlling the solubility, in particular, the dissolution properties in a neutral pH region, may be contained. Usable examples of the above additives acceptable as medicinal additives include, but are not limited to, those described according to application in Pharmaceutical Excipients Dictionary 2016 Edition (edited by Japan Pharmaceutical Excipients Council, Yakuji Nippo, Ltd.). It should be noted that these additives may be redundantly classified into a plurality of applications.

The plasticizer is not necessarily limited to specific substances shown in the above Pharmaceutical Excipients Dictionary, and is not particularly limited as long as it can be used in medicines or food compositions and can be added to impart flexibility to the capsule film. Suitable substances are those which generally have a molecular weight (Mw) of 100 to 20,000 and have one or more hydrophilic groups, such as hydroxyl groups, ester groups, or amino groups, in each molecule. Examples include dioctyl adipate, polyester adipate, epoxidized soybean oil, epoxyhexahydrophthalic acid diesters, kaolin, triethyl citrate, glycerin, glycerin fatty acid esters, sesame oil, dimethylpolysiloxane -silicon dioxide mixtures, D-sorbitol, medium-chain fatty acid triglyceride, corn starch-derived sugar alcohol liquid, triacetin, concentrated glycerin, castor oil, phytosterol, diethyl phthalate, dioctyl phthalate, dibutyl phthalate, butyl phthalyl butyl glycolate, propylene glycol, polyoxyethylene(105)polyoxypropylene(5)glycol, polysorbate 80, isopropyl macrogol myristate, cottonseed oil-soybean oil mixtures, glycerin monostearate, isopropyl linoleate, polyethylene glycols of various molecular weights (macrogols 400, 600, 1500, 4000, 6000), and so on. Polyethylene glycol is particularly preferred from the standpoint of having excellent compatibility and imparting high gloss. The weight-average molecular weight of the polyethylene glycol is not particularly limited, but is preferably 200 to 35000 from the standpoint of imparting high gloss.

The surfactant (also referred to as emulsifying agent) is used as a solubilizing agent, suspending agent, emulsifying agent, dispersing agent, dissolution aid, stabilizing agent or the like. Specific examples include benzalkonium chloride, benzethonium chloride polyoxyethylene (40)monostearate (polyoxyl 40 stearate^{∗}), sorbitan sesquioleate (sorbitan sesquioleate^{∗}), polyoxyethylene(20)sorbitan monooleate (polysorbate 80^{∗}), glyceryl monostearate (glycerin monostearate^{∗}), sodium lauryl sulfate, polyoxyethylene lauryl ether (lauromacrogol^{∗}) and so on (^{∗}: notation in Japanese Pharmacopoeia). Other examples include sodium alkylbenzene sulfonate, sucrose fatty acid esters, polyethylene glycol monooleate, polyethylene glycol dioleate, propylene glycol fatty acid esters (prooleic glycol monostearate), polyoxyethylene hydrogenated castor oil, polyoxyethylene glycerin monostearate, polyoxyethylene (160)polyoxypropylene(30)glycol, polyoxyethylene nonylphenyl ethers, and so on.

The hard capsule film may further contain a lubricant, a metal sequestering agent, a coloring agent, a light-shielding agent, a binder or the like in an amount of up to about 5% by mass. Examples of the metal sequestering agent can include ethylenediaminetetraacetic acid, acetic acid, boric acid, citric acid, gluconic acid, lactic acid, phosphoric acid, tartaric acid, or salts thereof, metaphosphates, dihydroxyethylglycine, lecithin, β-cyclodextrin, or combinations thereof.

The lubricant is not particularly limited as long as it can be used in medicines or food compositions. Examples can include calcium stearate, magnesium stearate, sodium stearyl fumarate, carnauba wax, starch, sucrose fatty acid esters, light anhydrous silicic acid, macrogol, talc, hydrogenated vegetable oils and so on.

Examples of the metal sequestering agent include ethylenediaminetetraacetic acid, acetic acid, boric acid, citric acid, gluconic acid, lactic acid, phosphoric acid, tartaric acid, or salts thereof, metaphosphates, dihydroxyethylglycine, lecithin, β-cyclodextrin, or combinations thereof.

The coloring agent and the light-shielding agent are not particularly limited as long as they can be used in medicines or food compositions. Examples of the coloring agent can include gambir tannin powder, turmeric extract, methylrosaniline chloride, yellow iron oxide, yellow ferric oxide, Opaspray K-1-24904, orange essence, brown iron oxide, carbon black, caramel, carmine, carotene liquid, β-carotene, photosensitizer 201, licorice extract, gold leaf, Sasa veitchii extract, black iron oxide, light anhydrous silicic acid, Daemonorops draco, zinc oxide, titanium oxide, iron sesquioxide, disazo yellow, Food Blue No. 1 and its aluminum lake, Food Blue No. 2 and its aluminum lake, Food Yellow No. 4 and its aluminum lake, Food Yellow No. 5 and its aluminum lake, Food Green No. 3 and its aluminum lake, Food Red No. 2 and its aluminum lake, Food Red No. 3 and its aluminum lake, Food Red No. 102 and its aluminum lake, Food Red No. 104 and its aluminum lake, Food Red No. 105 and its aluminum lake, Food Red No. 1 06 and its aluminum lake, sodium hydroxide, talc, sodium copper chlorophyllin, copper chlorophyll, hull-less barley green tea extract powder, hull-less barley green tea extract, phenol red, sodium fluorescein, d-borneol, malachite green, octyldodecyl myristate, methylene blue, medicinal carbon, riboflavin butyrate, riboflavin, green tea powder, ammonium manganese phosphate, sodium riboflavin phosphat, rose oil, turmeric color, chlorophyll, carminic acid color, Food Red No. 40 and its aluminum lake, water-soluble annatto, sodium iron chlorophyllin, dunaliella carotene, capsicum color, carrot carotene, potassium norbixin, sodium norbixin, palm oil carotene, beet red, grape pericarp color, black currant color, monascus color, safflower red color, safflower yellow color, marigold color, sodium riboflavin phosphate, madder color, alkanet color, aluminum, sweet potato carotene, shrimp color, krill color, orange color, cacao color, cacao carbon black, Japanese persimmon color, crayfish color, carob germ color, fish scale foil, silver, kusagi color, gardenia blue, gardenia red, gardenia yellow, kooroo color, chlorophylline, kaoliang color, bone carbon black, bamboo grass color, shea nut color, shikon color, sandalwood red, vegetable carbon black, sappan color, spirulina color, onion color, tamarind color, corn color, tomato color, peanut color, phaffia color, pecan nut color, monascus yellow, powdered annatto, Haematococcus algae color, purple sweet potato color, purple corn color, purple yam color, vegetable oil soot color, lac color, rutin, enju extract, buckwheat whole-plant extract, logwood color, red cabbage color, red rice color, red radish color, adzuki bean color, Hydrangea leaves extract, sepia color, uguisukagura color, elderberry color, olive tea, cowberry color, gooseberry color, cranberry color, salmonberry color, strawberry color, dark sweet cherry color, cherry color, thimbleberry color, European dewberry color, pineapple juice, black huckleberry color, grape juice color, black currant color, blackberry color, plum color, blueberry color, berry juice, boysenberry color, whortleberry color, mulberry color, morello cherry color, raspberry color, red currant color, lemon juice, loganberry color, powdered chlorella, cocoa, saffron color, beefsteak plant color, chicory color, laver color, hibiscus color, malt extract, paprika, beet red juice, carrot juice, and so on.

Examples of the light-shielding agent can include titanium oxide, calcium compounds, iron sesquioxide, yellow ferric oxide, black iron oxide, Food Blue No. 1 aluminum lake, Food Blue No. 2 aluminum lake, Food Yellow No. 4 aluminum lake, Food Yellow No. 5 aluminum lake, Food Green No. 3 aluminum lake, Food Red No. 2 aluminum lake, Food Red No. 3 aluminum lake, Food Red No. 102 aluminum lake, Food Red No. 104 aluminum lake, Food Red No. 105 aluminum lake, Food Red No. 106 aluminum lake, and Food Red No. 40 aluminum lake.

In order to prevent degradation of the content caused by ultraviolet rays or the like, titanium oxide and/or a calcium compound, in particular, may be added as a light-shielding agent to the hard capsule film. Examples of the calcium-containing compound include inorganic calcium salts such as calcium carbonate, and calcium hydrogen carbonate, calcium hydroxide, calcium hydroxide, calcium complexes such as dolomite and hydroxyapatite, and other calcium element-containing compounds.

Specific examples of the hard capsule can include gelatin capsules; gelatin capsules to which polyethylene glycol has been added (Japanese Unexamined Patent Application Publication No. H11-049667, Japanese Unexamined Patent Application Publication No. 2004-035569, Japanese Unexamined Patent Application Publication No. H11-060473 and so on); hydroxypropylmethylcellulose capsules (Japanese Unexamined Patent Application Publication No. 2000-136126, Japanese Unexamined Patent Application Publication No. 2000-202003, Japanese Unexamined Patent Application Publication No. 2000-237284, Japanese Unexamined Patent Application Publication No. 2000-297102, WO2005/113010, Japanese Translation of PCT International Application Publication No. 2009-524573, WO2008/084823, Japanese Unexamined Patent Application Publication No. 2010-202550, WO2018/008660, WO2018/105339, WO2019/013260 and so on); capsules using an enteric cellulose compound such as hydroxypropylmethylcellulose phthalate, hydroxypropylmethylcellulose acetate succinate, carboxymethylethylcellulose or cellulose acetate phthalate as a base (Japanese Unexamined Patent Application Publication No. 2006-16372 and so on), pullulan capsules (Japanese Patent Application No. H11-11665 and so on); enteric capsules composed of a film containing a water-soluble salt of alginic acid and at least one selected from the group consisting of gelatin, agar and curdlan (Japanese Unexamined Patent Application Publication No. 2009-196961 and so on); capsules containing PVA and/or a PVA copolymer (Japanese Unexamined Patent Application Publication No. 2001-170137, WO2009/125483, WO2009/125485, WO2018/008660 and so on); enteric hard capsules composed of a film (a) containing a first component and a second component, or (b) containing a first component and a second component and further containing at least one component from among a third component and a fourth component, wherein the first component is methylcellulose and/or hydroxypropylmethylcellulose with a viscosity value of 6 mPa·s or higher, the second component is an enteric methacrylic acid copolymer, the third component is a water-insoluble alkyl (meth)acrylate ester copolymer and/or ethylcellulose, and the fourth component is at least one selected from the group consisting of plasticizers and surfactants that are acceptable as pharmaceutical and food additives, and so on.

### 2. Method for filling content into hard capsule and sealing it

Filling of the content into the hard capsule can be conducted using a capsule filling machine known per se, such as a fully automatic capsule filling machine (model name: F-Labo/5/40/80/100/150/JCF/FUNFIL, manufactured by Qualicaps Co., Ltd.), a capsule sealing machine (model name: S-15/125, manufactured by Qualicaps Co., Ltd.) or the like. The body portion and cap portion of the thus obtained hard capsule are, after filling a content into the body portion, joined to each other by covering the body portion with the cap portion to fit them together. Then, when necessary, the filled capsule is tamper-proofed by using a suitable technique to seal the seam. Typically, a sealing or banding technique may be used, and these techniques are well-known to those skilled in the art in the field of capsules. As a specific example, the fitted portion can be sealed by applying a sealing agent of a polymer solution once or a plurality of times, preferably once or twice, to a surface of the body portion and a surface of the cap portion in a circumferential direction of the body portion and the cap portion at a certain width with an end edge of the cap portion in the middle.

When the capsule is sealed, the band seal preparation liquid can be generally used at room temperature or under warming. From the standpoint of preventing liquid leakage from the hard capsule, the sealing can be preferably conducted in a range of about 23 to 60°C. It is preferred that a band seal preparation liquid containing gelatin is warmed to about 45 to 60°C. For a band seal preparation liquid containing a cellulose compound, it is preferred to use a band seal preparation liquid in a temperature range of 15 to 45°C, preferably about 23 to 35°C, more preferably about 25 to 35°C. It should be noted that the temperature of the seal preparation liquid can be adjusted with a method known per se such as a panel heater or hot- water heater but it is preferred to adjust the temperature using a circulating hot-water heater or a seal-pan unit of the above-mentioned integrated capsule filling and sealing machine which has been converted to a circulating hot-water heater type, for example, because the temperature width can be delicately adjusted.

In this description, a product of a hard capsule filled with a content and sealed with a band seal is referred to as "hard capsule formulation." Also, a method for filling a hard capsule with a content and sealing it with a band seal is also a method for preparing a hard capsule formulation including sealing a hard capsule with a band seal.

The content that is filled into the hard capsule is not limited, but one component or two or more components selected, for example, from nutritional fortification healthcare agents, antipyretic, analgesic and anti-inflammatory agents, psychotropic agents, anxiolytic agents, antidepressants, hypnosedatives, anticonvulsive agents, central nervous system agents, brain metabolism-improving agents, brain circulation-improving agents, antiepileptic agents, sympathomimetic stimulants, gastrointestinal agents, antacids, anti-ulcer agents, antitussive and expectorant agents, antiemetic agents, anapnoics, bronchodilators, anti-allergic agents, agents for dental and oral use, antihistamines, cardiotonic agents, agents for arrhythmia, diuretic agents, hypotensive agents, vasoconstrictive agents, coronary vasodilators, peripheral vasodilators, agents for hyperlipidemia, cholagogues, antibiotics, chemotherapeutic agents, agents for diabetes, agents for osteoporosis, antirheumatic agents, skeletal muscle relaxants, spasmolytic agents, hormonal agents, alkaloidal narcotics, sulfa drugs, arthrifuges, anticoagulant agents, and antineoplastic agent and so on is/are filled. It should be noted that these pharmaceutically effective components are not particularly limited and may include a variety of known components. These components may be used alone or as a compound drug with other components. Also, these components are filled in a fixed known amount as appropriate based on the condition, age and so on of the patient.

Examples of the nutritional fortification healthcare agents include vitamins such as vitamin A, vitamin D, vitamin E (e.g., d-α-tocopherol acetate), vitamin B1 (e.g., dibenzoyl thiamine, fursultiamine hydrochloride), vitamin B2 (e.g., riboflavin butyrate), vitamin B6 (e.g., pyridoxine hydrochloride), vitamin C (e.g., ascorbic acid, sodium L-ascorbate), and vitamin B12 (e.g., hydroxocobalamin acetate, cyanocobalamin); minerals such as calcium, magnesium, and iron; proteins; amino acids; oligosaccharides; crude drugs and so on.

Examples of the antipyretic, analgesic and anti-inflammatory agents include aspirin, acetaminophen, ethenzamide, ibuprofen, diphenhydramine hydrochloride, chlorpheniramine dl-maleate, dihydrocodeine phosphate, noscapine, methylephedrine hydrochloride, phenylpropanolamine hydrochloride, caffeine, caffeine anhydride, serrapeptase, lysozyme chloride, tolfenamic acid, mefenamic acid, diclofenac sodium, flufenamic acid, salicylamide, aminopyrine, ketoprofen, indomethacin, bucolome, pentazocine and so on.

Healthy foods (including foods for specified health use or nutritional supplements, such as fucoidan, heme iron, polyphenols) that may be filled in the hard capsule include peptides and amino acids (e.g., royal jelly, ornithine, citrulline, aminolevulinic acid, black vinegar, or hydrophobic amino acids such as methionine, valine, leucine and isoleucine), proteins (e.g., milk proteins such as lactoferrin, collagen and placenta), glycoproteins, enzyme-fermented foods (e.g., nattokinase), coenzymes (e.g., coenzyme Q10), vitamins (e.g., β-carotene), minerals, viable bacteria (e.g., yeasts, Lactobacillus and bifidobacteria), plant extracts (e.g., crude drugs, and herbs such as turmeric extract, carrot extract, Japanese plum extract, ginkgo leaf extract, blueberry extract and Rubus suavissimus extract), natural organic substances such as propolis, or any combination thereof.

However, the content is not limited to these.

Attaching a band seal makes it possible to prevent or find opening before ingestion or exchange of the contents. Further, sealing the hard capsule with a band seal preparation liquid containing the tags makes it possible to prevent counterfeit of the hard capsule formulation and to determine the origin of the hard capsule formulation because the presence or absence of the tags or the identifier the tags have can be detected.

Therefore, the above-mentioned method for sealing a hard capsule can be also regarded as a method for preventing counterfeit of a hard capsule formulation including filling a hard capsule with a content followed by fitting the cap portion and the body portion thereof together, applying a band seal preparation liquid as exemplified in the above section 1. to a formed fitted portion, and drying the band seal preparation liquid to seal the hard capsule.

### 3. Detection of tags

The tags or the identifier attached to the tags can be detected by irradiating the hard capsule formulation with light in a predetermined wavelength region and using reflected light or fluorescence emitted from the tags as an indicator. A light source for irradiation of light is not limited as long as it can emit light in a predetermined wavelength region. For example, a blue light or black light can be used as the light source.

Examples of the light in a predetermined wavelength region can include light in a visible region, or light in an ultraviolet region. For the light in a predetermined wavelength region, the explanation described in the above section 1. is incorporated here.

The reflected light or fluorescence derived from the tags can be confirmed with naked eyes, macro (closeup) observation, a magnifier, a stereomicroscope, a fluorescent microscope or the like. When the tags are irradiated with light in a visible region, the light is reflected by the detection substance applied to the tags and reflected light is obtained. Also, when the detection substance contains a fluorescent component, fluorescence is obtained when the tags are irradiated with light in an ultraviolet region. When fluorescence is detected, a bandpass filter can be used to selectively observe fluorescence desired to be detected.

At the time of detection, when only the presence or absence of the tags is detected, the detection can be made with naked eyes, by macro (closeup) observation, or at a low magnification of about 2 to 10 times. When an identifier is detected, the identifier can be read by enlarging it about 40 to 100 times.

Also, when the identifier is a one-dimensional barcode, two-dimensional barcode, or a rugate type structure, it can be read with a dedicated reader after enlargement.

In the methods described in Patent Literature 1, Patent Literature 2 and Non-Patent Literature 2, identification of the tracer particles is complicated because the drug must be once dissolved to separate the tracer particles from other components. Also, the drugs to be ingested must be wasted in order to identify the tracer. However, in this embodiment, detection is easy because the tags can be detected without separating them from the hard capsule formulation. Thus, even patients can detect the tags without reducing the medicines to be ingested.

Thus, the method for detecting tags can also be regarded as a method for tracking a hard capsule formulation including the steps of irradiating a hard capsule formulation sealed with a band seal preparation liquid containing tags that enable a product to be identified with light in a predetermined wavelength region, and detecting the tags or an identifier.

### Examples

While examples are shown below to describe the present invention in more detail, the present invention is not construed as being limited to the examples.

### I. Example 1

### 1. Fluorescent pigment

As a fluorescent pigment, Fluorescent Pigment Blue manufactured by Kurachi Co. Ltd. was used.

### 2. Preparation of band seal preparation liquid

An aqueous solution containing 22 wt% of gelatin and 2 wt% of polysorbate 80 was prepared as a clear band seal preparation liquid. Specifically, 228 g of water was added to 66 g of gelatin to swell the gelatin, followed by warming at 53°C to dissolve the gelatin. After the gelatin was completely dissolved, 6 g of polysorbate 80 was added, and the mixture was stirred with a spatula to prepare a clear band seal preparation liquid. A 10 wt% aqueous solution of Blue No. 1 was added to this clear band seal preparation liquid so as to be 1 wt% to prepare a blue band seal liquid.

Each band seal preparation liquid was maintained at 53 to 55°C when used.

### 3. Sealing of capsule

As transparent capsules, size 2 gelatin capsules manufactured by Qualicaps Co., Ltd. (product number: A7122L) were prepared. The cap portion and body portion of each capsule were fitted together, and the band seal preparation liquid was applied across the fitted portion and dried after the band sealed portion was sprinkled with a minute amount of the fluorescent pigment. A tabletop band sealing machine (LAB-TOP, manufactured by Schaefer Technologies) was used as a sealing machine, and sealing was performed with the band seal preparation liquid in the seal pan heated at 53°C.

### 4. Detection of fluorescent pigment

### 4.1. Fluorescent observation conditions

Using a system microscope (Olympus BX53F), irradiation of visible light in a blue region between 460 nm and 495 nm (B excitation) was performed and its fluorescence was observed. At the time of B excitation, an absorption filter 510IF was used. The observation was made at a magnification of 40 times.

### 4.2. Low-magnification observation of fluorescent pigment in band seal

A band seal was applied to the transparent capsule with the blue band seal preparation liquid containing the fluorescent pigment, and observation was made under a magnifier under B excitation.

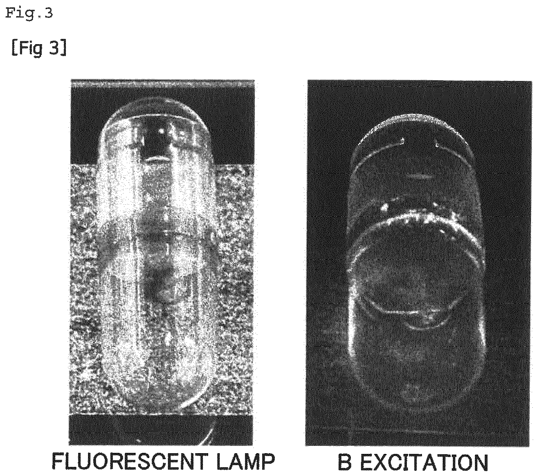

The results are shown in FIG. 3. The left-hand side of FIG. 3 shows the appearance of the capsule under a normal fluorescent lamp. The appearance of the capsule under B excitation is shown on the right-hand side of FIG. 3. The fluorescent pigment in the band seal was perceptible as pale fluorescence.

This demonstrates that the fluorescent pigment in the band seal can be detected by irradiation of light in an ultraviolet region.

### 4.3. High-magnification observation of fluorescent pigment in band seal

A band seal was applied to a transparent capsule with the blue band seal preparation liquid containing the fluorescent pigment, and observation of the fluorescent pigment was made using a fluorescent microscope under B excitation.

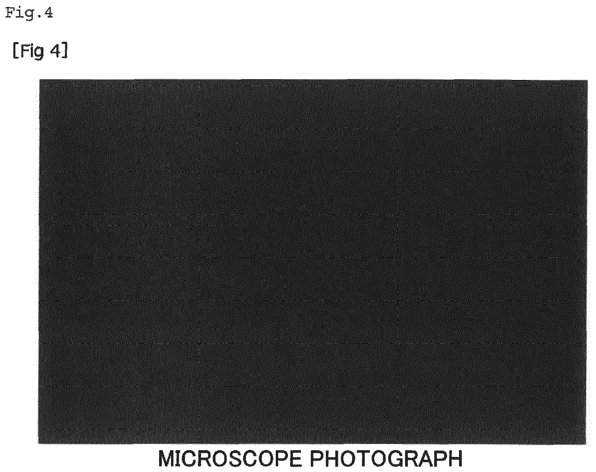

The results are shown in FIG. 4.

### II. Example 2

While examples are shown below to describe the present invention in more detail, the present invention is not construed as being limited to the examples.

### 1. Fluorescent pigment

As a fluorescent pigment, Fluorescent Pigment Blue manufactured by Kurachi Co. Ltd. was used.

### 2. Preparation of band seal preparation liquid

An aqueous solution containing 16 wt% of HPMC and 50.4 wt% of dehydrated ethanol was prepared as a clear band seal preparation liquid. Specifically, after 151.2 g of dehydrated ethanol was added to 48.0 g of HPMC to disperse the HPMC, 100.8 g of ion-exchange water was added to dissolve the HPMC completely to prepare a clear band seal preparation liquid. A 10 wt% aqueous solution of Blue No. 1 was added to this clear band seal preparation liquid so as to be 1 wt% to prepare a blue band seal liquid.

The fluorescent pigment as described in section 1. was added to the band seal preparation liquid so as to be about 0.2 wt%, and the mixture was stirred with a spatula to prepare a band seal preparation liquid with the fluorescent pigment dispersed therein. Each band seal preparation liquid was maintained at 20 to 30°C when used.

### 3. Sealing of capsule

As white capsules, size 2 HPMC capsules manufactured by Qualicaps Co., Ltd. (product number:C6149E) were prepared. The white capsules contain titanium dioxide. The cap portion and body portion of each capsule were fitted together, and the band seal preparation liquid was applied across the fitted portion and dried. A tabletop band sealing machine (LAB-TOP, manufactured by Schaefer Technologies) was used as a sealing machine, and sealing was performed with the band seal preparation liquid in the seal pan heated at 25°C.

### 4. Detection of fluorescent pigment

### 4.1. Fluorescent observation conditions

Using a system microscope (Olympus BX53F), irradiation of visible light in a blue region between 460 nm to 495 nm (B excitation) was performed and its fluorescence was observed. At the time of B excitation, an absorption filter 510IF was used. The observation was made at a magnification of 40 times.

### 4.2. Low-magnification observation of fluorescent pigment in band seal

A band seal was applied to the white capsule with the blue band seal preparation liquid containing the fluorescent pigment, and observation was made under a magnifier under B excitation.

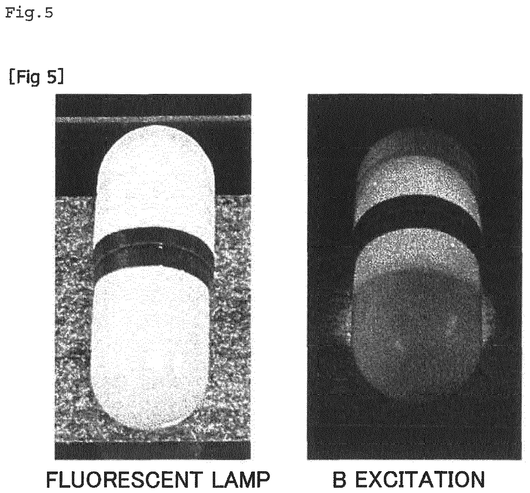

The results are shown in FIG. 5. The left-hand side of FIG. 5 shows the appearance of the capsule under a normal fluorescent lamp. The appearance of the capsule under B excitation is shown on the right-hand side of FIG. 5. In the observation of all band seals and capsules, the fluorescent pigment in the band seal was perceptible as pale fluorescence.

### 4.3. High-magnification observation of fluorescent pigment in band seal

A band seal was applied to the white capsule with the blue band seal preparation liquid containing the fluorescent pigment, and observation of the fluorescent pigment was made using a fluorescent microscope under B excitation.

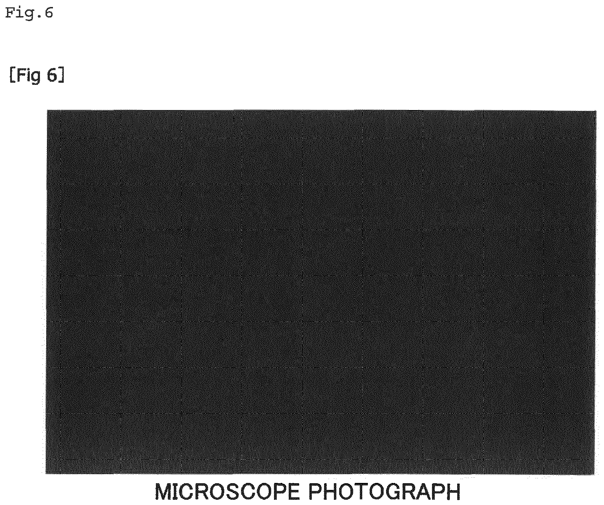

The results are shown in FIG. 6.

## Claims

1. A hard capsule formulation sealed with a band seal preparation liquid containing tags that enable a product to be identified.

2. The hard capsule formulation according to Claim 1, wherein the tags have a length of 400 µm or less.

3. The hard capsule formulation according to Claim 1 or 2, wherein the tags have an identifier for identifying the product.

4. The hard capsule formulation according to Claim 3, wherein the identifier can be read when irradiated with light in a predetermined wavelength region.

5. The hard capsule formulation according to Claim 3 or 4, wherein the identifier is at least one selected from the group consisting of letters, colors, figures, trademarks, emblems, one-dimensional barcodes, two-dimensional barcodes, and rugate type structures.

6. The hard capsule formulation according to any one of Claims 1 to 5, wherein a film of the hard capsule contains a base and a component other than the base,
the base being at least one selected from the group consisting of gelatin, cellulose compounds, polyvinyl alcohol, polyvinyl alcohol copolymers and pullulan,
the component other than the base being at least one selected from gelling agents, gelation aids, plasticizers, lubricants, metal sequestering agents, coloring agents, light-shielding agents, and residual moisture.

7. A band seal preparation liquid for sealing a hard capsule, the band seal preparation liquid containing tags that enable a product to be identified.

8. A method for preparing a hard capsule formulation, comprising filling a hard capsule with a content, followed by fitting a cap portion and a body portion thereof together, applying a band seal preparation liquid according to Claim 7 to a formed fitted portion, and drying the band seal preparation liquid to seal the hard capsule.

9. A method for preventing counterfeit of a hard capsule formulation, comprising filling a hard capsule with a content, followed by fitting a cap portion and a body portion thereof together, applying a band seal preparation liquid according to Claim 7 to a formed fitted portion, and drying the band seal preparation liquid to seal the hard capsule.

10. A method for tracking a hard capsule formulation, comprising the step of irradiating a hard capsule formulation according to any one of Claims 1 to 6 with light in a predetermined wavelength region to detect the tags or identifier.
